# EUROPEAN PATENT SPECIFICATION

(11) **EP 1 909 867 B1**
(45) Date of publication and mention of the grant of the patent: **30.04.2014**
(21) Application number: 06779712.6
(22) Date of filing: 14.06.2006
(51) Int. Cl.: A61M 3/02, A61F 5/00

(54) **SANITARY INSTRUMENT FOR THE IRRIGATION OF ANATOMIC CANALS, ESPECIALLY THE RECTAL CANAL**
SANITÄRINSTRUMENT ZUR IRRIGATION VON ANATOMISCHEN KANÄLEN, INSBESONDERE DEM REKTALKANAL
INSTRUMENT MÉDICAL POUR IRRIGUER DES CONDUITS ANATOMIQUES, NOTAMMENT LE CANAL RECTAL

(30) Priority: 14.06.2005 IT MO20050147
(43) Date of publication of application: 16.04.2008
(73) Proprietor: Lameplast SPA, Frazione Rovereto sul Secchia (MO) (IT)
(72) Inventor: FONTANA, Antonio, I-41012 Carpi (MO) (IT)
(74) Representative: Brunacci, Marco
(86) International application number: PCT/IB2006/001576
(87) International publication number: WO 2006/134464

(56) References cited:
- EP-A- 0 139 855
- US-A- 4 248 228
- US-A- 5 843 043
- US-A1- 2003 088 217

## Description

### Technical Field

The present invention refers to a sanitary instrument for the irrigation of anatomic canals, especially the rectal canal.

### Background art

Sanitary instruments are known for irrigating anatomic canals, especially the rectal canal, with washing fluids, creams, soothing ointments or other pharmaceutical fluids.

Such instruments are, generally, of the disposable type and packed already prefilled with a pre-determined dose of fluid.

The known instruments comprise a tank containing a pre-established quantity of fluid and which features an opening with which an irrigation cannula is associated for insertion into the anatomic canal, especially the rectal section of the intestine.

The sides of the tank are deformable in elastic field and can be made, for example, of rubber or other elastomer material, or else have a bellows configuration.

The cannula consists of a tubular element with an axial end associated with the tank opening and which, on the opposite axial end, features a dispensing mouth. After inserting at least one section of the cannula into an anatomic canal, especially the rectal canal of a patient, on the sides of the tank a crushing pressure is exercised from outside as a result of which the fluid this contains is pushed through the cannula and flows through its dispensing mouth.

These sanitary instruments are not without drawbacks, among which must be mentioned the fact that, because the fluid dispensing mouth is defined at the axial end of the cannula, meaning at the bottom surface of the cannula end which is inserted in the anatomic canal, the flow of dispensed fluid runs in an axial direction along the section of the anatomic canal downstream the cannula, thus preventing correct irrigation of the canal side walls and, in particular, of the section of the canal in which the cannula is inserted.

To overcome such drawback, sanitary instruments are known that feature dispensing mouths defined on the side walls of the section of the cannula inserted in an anatomic canal.

When a crushing pressure is applied on the walls of the tank the fluid flow runs crossways to the longitudinal axis of the cannula, irrigating the side walls of the anatomic canal section inside which the cannula is inserted.

The particular configuration of the tank does not however allow correct dosage of the fluid inside the anatomic canal and - a further drawback of these known instruments does not ensure complete dispensing of the fluid contained inside the tank,

Inside the tank in fact, fluid residues remain due both to return flows caused by the expansion of the tank when the crushing pressure stops and to the formation of stagnant fluid between the folds of the bellows.

Other kinds of dispenser instruments are disclosed by the patent documents EP 0 139 855, US 4,248,228, US 5,843,043 and US 2003/088217.

### Disclosure of the invention

The principal aim of this invention is to eliminate the drawback complained of above relating to known sanitary instruments by excogitating a sanitary instrument for the irrigation of anatomic canals, especially the rectal canal, that permits completely irrigating the walls of the anatomic canal, especially the rectal canal, and distributing the fluid correctly on them.

A further purpose of this invention is to permit the complete dispensing of the fluid and a void the formation of residues inside the tank.

As part of such technical aim, another purpose of the present invention is to achieve the previous aims with a simple structure, of relatively practical implementation, safe use and effective operation, as well as of a relatively low cost.

This aim and these purposes are all achieved by this sanitary instrument for the irrigation of anatomic canals, especially the rectal canal, according to claim 1.

### Brief Description of the Drawings

Further characteristics and advantages of this invention will appear even more evident from the detailed description of a preferred, but not exclusive, form of embodiment of a sanitary instrument for the irrigation of anatomic canals, especially the rectal canal, illustrated by way of non limiting example in the accompanying drawings, wherein:

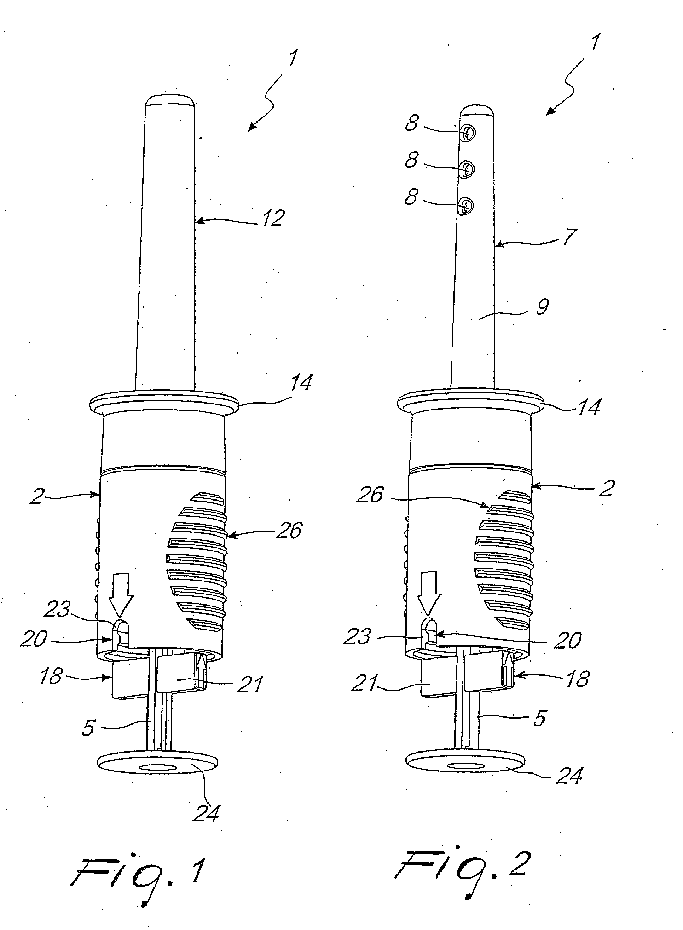

figure 1 is an axonometric view of the sanitary instrument, according to the invention;
figure 2 is an axonometric view of the sanitary instrument, according to the invention and without the cover cap;

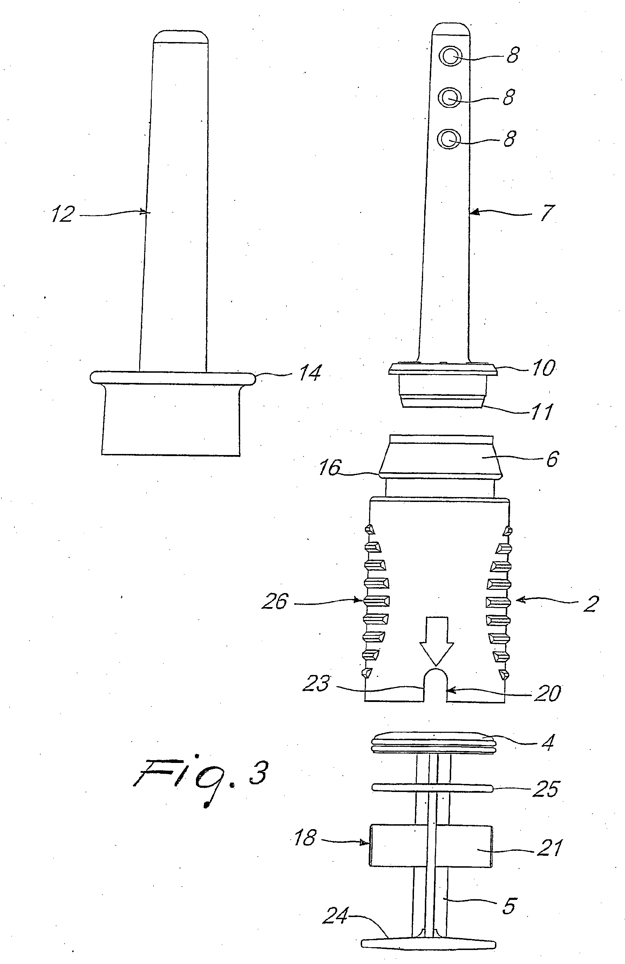

figure 3 is a side exploded view of the sanitary instrument, according to the invention;

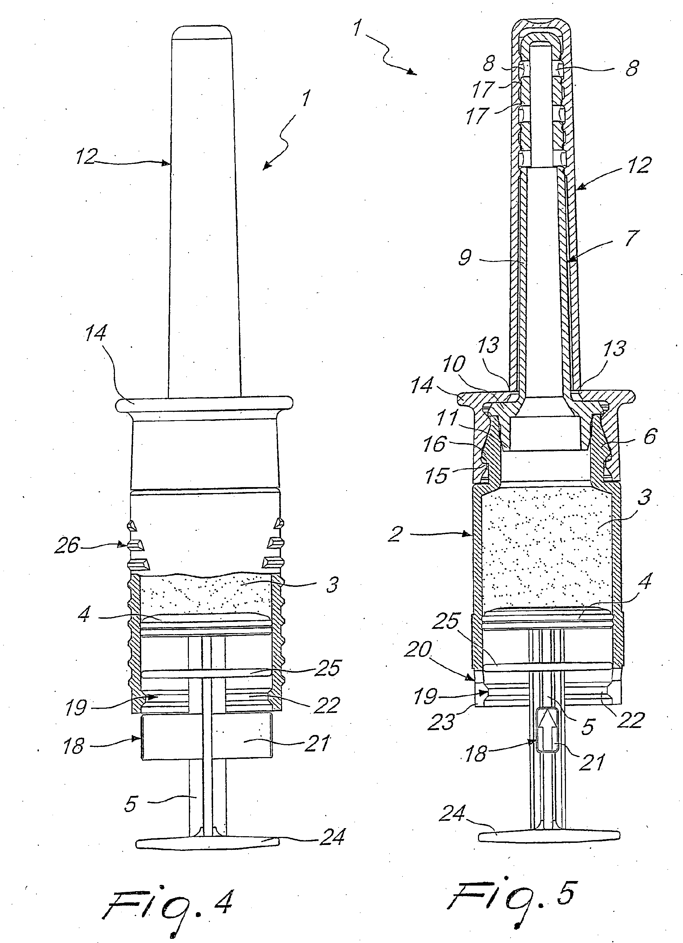

figure 4 is a partially cross-section side view of the sanitary instrument with the piston in retention configuration, according to the invention;
figure 5 is a side cross-section view of the sanitary instruments with the piston in retention configuration, according to the invention;

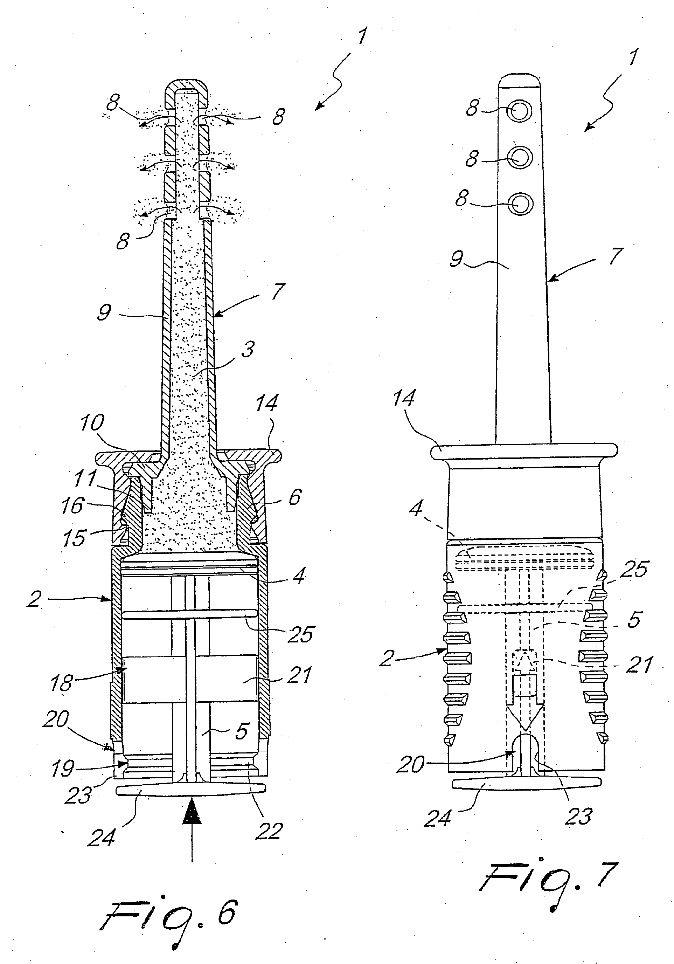

figure 6 is a side cross-section view of the sanitary instrument with the piston in release configuration, according to the invention;
figure 7 is a side view of the sanitary instrument with the piston in release configuration, according to the invention;

### Ways of carrying out the Invention

With special reference to such figures, a sanitary instrument for the irrigation of anatomic canals, especially the rectal canal of a patient has been generally designated by reference numeral 1.

The instrument 1 comprises a body 2 of substantially cylinder shape and hollow so as to contain a fluid 3, inside which a piston 4 is housed in a substantially sealed manner and sliding axially.

An operating rod 5 of the piston 4 moves through an open end of the body 2. The end of the body 2 opposite the open end features a beak 6 to which is associated a cannula 7, one section of which at least can be fitted in an anatomic canal, especially in the rectal canal of a patient.

The section of the cannula 7 that can be fitted features, on the side, at least one dispensing mouth 8 of fluid 3 for the irrigation of the rectal canal.

The particular side position of the dispensing mouth 8 permits the dispensing of the fluid 3 in a direction crossways to the longitudinal axis of the section of the fitted cannula 7, and consequently in a direct way to the walls of the patient's rectal canal.

In particular, the cannula 7 comprises a tubular element 9 which is connected by its open end to the beak 6. This end consists of a ring-shaped collar 10 fast on to the edge of the beak 6 and from which extends a tubular appendix 11 which can be sealed into the beak 6.

The end of the tubular element 9 opposite the end fastened to the beak 6 defines the section of cannula 7 that can be fitted and is closed at its base surface. Advantageously, the section of cannula 7 that can be fitted features a number of pairs of dispensing mouths 8, distributed at a distance the one from the other in the longitudinal direction of such section, and with each pair featuring dispensing mouths 8 defined exactly opposite one another.

The instrument 1 also comprises a cap 12 for covering cannula 7 and designed to prevent accidental exits of the fluid 3 from the dispensing mouths 8 before application to the patient's rectal duct.

The cap 12 is associated in a removable way along a weakening line 13 with a flange 14 associated integrally with beak 6. In particular, the weakening line 13 consists of a series of breakable bridges.

The flange 14 features a projection 15 which engages undercut fashion with the raised section 16 defined on the outer surface of the beak 6, securing this to the body 2.

At the same time, the ring-shaped collar 10 of the cannula 7 is held between the edge of the beak 6 and the flange 14 which therefore acts in such a way as to secure the cannula 7 to the body 6.

The cap 12 comprises at least a pair of seal rings 17 defined on its inner side surface and able to delimit the dispensing mouth 8. In particular, a series of pairs of seal rings 17 are fitted, arranged so that each delimits each corresponding pair of the series of pairs of dispensing mouths 8.

The instrument 1 also comprises means for temporarily retaining the piston 4 able to prevent it sliding axially from the open end of the body 2 to the beak 6, with consequent accidental exit of the fluid 3 from the series of pairs of dispensing mouths 8.

The means for temporarily retaining comprise a retaining element 18, associated integrally with the rod 5 and which can be engaged with a stop element 19 defined close to the open end of body 2.

Disengagement means 20 are also provided for the retaining element 18 and the stop element 19 which can be started before the instrument 1 is used for irrigating the rectal canal.

In particular, the retaining element 18 comprises at least one wing 21 which extends crossways from rod 5 and the stop element 19 comprises a ring-shaped tooth 22 defined in centripetal overhang on the inner side surface of body 2.

The disengagement means feature at least one longitudinal cut 23, defined in the walls of the body 2 and which interrupts the continuity of the ring-shaped tooth 22.

In a retention configuration of the piston 4 the free end of wing 21 is in contact with the ring-shaped tooth 22, outside the body 2, and thus prevents the piston 4 from sliding towards the beak 6 and, consequently, prevents any pressure action of the piston 4 on the fluid 3 inside the body 2.

An axial rotation of the rod 5 permits achieving a release configuration of the piston 4, in which the free end of wing 21 is positioned at the longitudinal cut 23. In this configuration, the sliding of the piston 4 towards the beak 6 is made possible by fitting the wing inside the body 2, through the longitudinal cut 23. Furthermore, the ring-shaped tooth 22 prevents the piston 4 coming out through the open end of body 2.

In particular, there are two wings 21 arranged completely opposite each other and which can be aligned by rotation of the rod 5 and which can be fitted in two longitudinal cuts 23 respectively, defined on the side walls of body 2.

The rod 5 is fastened to an end of the piston 4 and, at the other end, features a grip 24 of substantially disc shape.

The wings 21 are fastened to the rod 5 between the piston 4 and the grip 24 and a seal disc 25 is placed in between the two wings 21 and the piston 4.

Usefully, the outer surface of body 2 features a succession of reliefs 26, distributed with substantially regular pitch and able to ensure a sound grip.

The operation of this invention is the following.

The body 2 of the sanitary instrument 1 is arranged with the cap 12 fitted and the flange 14 fitted onto the beak 6. The wings 21 are arranged externally at the open end of body 2 and the rod 5 is turned in retention configuration for piston 4.

The body 2 is also preliminarily filled with the fluid 3 that can be used to irrigate the patient's rectal canal.

Before use, the cap 12 is removed by intervening on this with traction and/or rotation with the consequent breakage of the weakening lines 13.

The section of the cannula 7 consisting of the tubular element 9 is partially fitted inside the patient's rectal canal.

A pressure on the grip 24 allows the piston 4 to slide from the open end of body 2 towards the beak 6.

The piston 4 therefore exercises a crushing pressure on the fluid 3, which runs through the beak 6, along the tubular element 9 and exits from the dispensing mouths 8, irrigating the walls of the rectal duct.

It has in fact been found that the described invention achieves the intended purposes and in particular the fact must be emphasised that the sanitary instrument permits completely irrigating the walls of the anatomic canal, especially the rectal canal, and correctly distributing the fluid on these.

This invention also permits the virtually complete dispensing of the fluid, avoiding the formation of residues inside the tank.

The absence is also assured of accidental fluid leaks while transporting the preliminarily filled sanitary instrument.

The invention thus conceived is susceptible of numerous modifications and variations, all of which falling within the scope of the inventive concept. Furthermore all the details can be replaced with others that are technically equivalent.

In practice, the materials used, as well as the shapes and dimensions, may be any according to requirements without because of this moving outside the protection scope of the following claims.

## Claims

1. Sanitary instrument (1) for the irrigation of anatomic canals, especially the rectal canal, comprising a substantially cylinder-shaped and hollow body (2) containing a fluid (3) which has an open end through which an operating rod (5) of a piston (4) moves, which is substantially housed in a sealed manner and sliding axially inside said body (2), and with the opposite end featuring a beak (6) with which is associated a cannula (7), at least a section of which is able to be inserted in an anatomic canal, especially in the rectal canal of a patient to irrigate this with said fluid (3), the cannula (7) being provided with at least one fluid dispensing mouth (8) which is defined on the side surface of said section, said instrument (1) further comprising means for temporarily retaining said piston (4) able to prevent it sliding axially from said open end of said body (2) to said beak (6), **characterized by** the fact that said means for temporarily retaining comprise a retaining element (18), associated integrally with said rod (5) and which can be engaged with a stop element (19) defined close to said open end of said body (2), disengagement means (20) being provided for said retaining element (18) and said stop element (19).

2. Instrument (1) according to claim 1, **characterized by** the fact that said cannula (7) comprises a tubular element (9) which has an open end and able to be associated with said beak (6) and the opposite end closed, said section being defined near said closed end.

3. Instrument (1) according to one or more of the preceding claims, **characterized by** the fact that said cannula (7) comprises at least one pair of said dispensing mouths (8), defined exactly opposite one another.

4. Instrument (1) according to claim 3, **characterized by** the fact that it comprises a series of said pairs (8) distributed at a distance the one from the other in the longitudinal direction of such section.

5. Instrument (1) according to one or more of the preceding claims, **characterized by** the fact that said retaining element (18) comprises at least one wing (21) which extends crossways from said rod (5).

6. Instrument (1) according to claim 5, **characterized by** the fact that said stop element (19) comprises a ring-shaped tooth (22) defined in centripetal overhang on the inner side surface of said body (2) and able to be in contact with the free end of said at least one wing (21).

7. Instrument (1) according to claim 6, **characterized by** the fact that said disengagement means (20) feature at least one longitudinal cut (23), defined in the walls of said body (2) and able to interrupt the continuity of said tooth (22), said rod (5) being able to turn from a retention configuration of said piston (4), in which the free end of said at least one wing (21) is in contact with said tooth (22), to a release configuration of said piston (4), in which the free end of said at least one wing (21) is positioned by said longitudinal cut (23).

8. Instrument (1) according to claim 7, **characterized by** the fact that said wings (21) are two arranged completely opposite each other, said cuts (23) being two completely opposite each other.

9. Instrument (1) according to one or more of the claims 5 to 8, **characterized by** the fact that said rod (5) is fastened to an end of said piston (4) and, at the other end, it features a grip (24) of substantially disc shape, said at least one wing (21) being placed in between said grip (24) and said piston (4).

10. Instrument (1) according to one or more of the preceding claims, **characterized by** the fact that it comprises a removable cap (12) for covering said cannula (7).

11. Instrument (1) according to claim 10, **characterized by** the fact that said cap (12) is associated in a removable way along a weakening line (13) with a flange (14) associated integrally with said beak (6).

12. Instrument (1) according to claim 10 or 11, **characterized by** the fact that said cap (12) comprises of at least one pair of seal rings (17) defined non its inner side surface and able to delimit said at least one dispensing nouth (8).

13. Instrument (1) according to one or more of the preceding claims, **characterized by** the fact that said body (2) features a succession of grip raised sections (26) on its outer surface.

## Patentansprüche

1. Sanitärinstrument (1) zur Spülung von Körperkanälen, insbesondere des Rektums, umfassend einen im Wesentlichen zylinderförmigen und hohlen, ein Fluid enthaltenden Körper (2), der ein offenes Ende aufweist, durch das sich eine Betätigungsstange (5) eines Kolbens (4) bewegt, der im Wesentlichen abgedichtet und axial gleitend in dem Körper (2) angeordnet ist und am gegenüberliegenden Ende eine Tülle (6) aufweist, die mit einer Kanüle (7) verbunden ist, wobei zumindest ein Abschnitt hiervon in einen Körperkanal, insbesondere in das Rektum eines Patienten zur Spülung desselben mit dem Fluid (3), eingeführt werden kann, wobei die Kanüle (7) zumindest eine Fluid-Abgabeöffnung (8) aufweist, die an der Seitenfläche des Abschnitts ausgebildet ist, wobei das Instrument (1) ferner Mittel zur zeitweisen Fixierung des Kolbens (4) aufweist, um ein axiales Verrutschen desselben vom offenen Ende des Körpers (2) in Richtung der Tülle (6) zu verhindern, **dadurch gekennzeichnet, dass** die Mittel zur zeitweisen Fixierung ein einstückig mit der Stange (5) verbundenes Halteelement (18), welches mit einem Anschlagelement (19) in Eingriff gebracht werden kann, das in der Nähe des offenen Endes des Körpers (2) ausgebildet ist, sowie Entkopplungsmittel (20) für das Halteleement (18) und das Anschlagelement (19) umfassen.

2. Instrument (1) gemäß Anspruch 1, **dadurch gekennzeichnet, dass** die Kanüle (7) ein rohrförmiges Element (9) umfasst, das ein offenes Ende aufweist und mit der Tülle (6) und dem gegenüberliegenden geschlossenen Ende verbunden werden kann, wobei dieser Abschnitt nahe dem geschlossenen Ende ausgebildet ist.

3. Instrument (1) gemäß einem oder mehreren der vorigen Ansprüche, **dadurch gekennzeichnet, dass** die Kanüle (7) zumindest ein Paar Abgabeöffnungen (8) umfasst, die genau gegenüber voneinander ausgebildet sind.

4. Instrument (1) gemäß Anspruch 3, **dadurch gekennzeichnet, dass** es eine Reihe der Paare (8) umfasst, die in Längsrichtung eines solchen Abschnitts beabstandet zueinander verteilt sind.

5. Instrument (1) gemäß einem oder mehreren der vorigen Ansprüche, **dadurch gekennzeichnet, dass** das Halteelement (18) zumindest einen Flügel (21) umfasst, der sich quer zur Stange (5) erstreckt.

6. Instrument (1) gemäß Anspruch 5, **dadurch gekennzeichnet, dass** das Anschlagelement (19) einen ringförmigen Zahn (22) umfasst, der zentripetal überstehend an der inneren Seitenfläche des Körpers (2) ausgebildet ist und in Anlage mit dem freien Ende des zumindest einen Flügels (21) gebracht werden kann.

7. Instrument (1) gemäß Anspruch 6, **dadurch gekennzeichnet, dass** die Entkopplungsmittel (20) zumindest eine in den Wänden des Körpers (2) ausgebildete Längsausnehmung (23) aufweisen, die die Durchgängigkeit des Zahns (22) unterbrechen kann, wobei die Stange (5) von einer Haltestellung des Kolbens (4), in der das freie Ende des zumindest einen Flügels (21) gegen den Zahn (22) anliegt, in eine Freigabestellung des Kolbens (4) gedreht werden kann, in der das freie Ende des zumindest einen Flügels (21) durch die Längsausnehmung (23) positioniert wird.

8. Instrument (1) gemäß Anspruch 7, **dadurch gekennzeichnet, dass** es sich bei den Flügeln (21) um zwei handelt, die exakt gegenüber voneinander angeordnet sind, wobei es sich bei den Ausnehmungen (23) um zwei handelt, die exakt gegenüber voneinander angeordnet sind.

9. Instrument (1) gemäß einem oder mehreren der Ansprüche 5 bis 8, **dadurch gekennzeichnet, dass** die Stange (5) an einem Ende des Kolbens (4) befestigt ist und am anderen Ende einen im wesentlichen scheibenförmigen Griff (24) aufweist, wobei der zumindest eine Flügel (21) zwischen dem Griff (24) und dem Kolben (4) angeordnet ist.

10. Instrument (1) gemäß einem oder mehreren der vorigen Ansprüche, **dadurch gekennzeichnet, dass** es eine abnehmbare Abdeckung (12) zur Abdeckung der Kanüle (7) umfasst.

11. Instrument (1) gemäß Anspruch 10, **dadurch gekennzeichnet, dass** die Abdeckung (12) entlang einer Sollbruchstelle (13) lösbar mit einem einstückig mit der Tülle (6) verbundenen Flansch (14) verbunden ist.

12. Instrument (1) gemäß Anspruch 10 oder 11, **dadurch gekennzeichnet, dass** die Abdeckung (12) zumindest ein Paar Dichtungsringe (17) umfasst, die an ihrer inneren Seitenfläche ausgebildet sind und die zumindest eine Abgabeöffnung (8) begrenzen können.

13. Instrument (1) gemäß einem oder mehreren der vorigen Ansprüche, **dadurch gekennzeichnet, dass** der Körper (2) an seiner Außenfläche eine Aufeinanderfolge von erhabenen Griffabschnitten (26) aufweist.

## Revendications

1. Instrument (1) médical pour l'irrigation de conduits anatomiques, notamment le canal rectal, comprenant un corps (2) creux et de forme sensiblement cylindrique qui contient un fluide (3) et qui présente une extrémité ouverte à travers laquelle se déplace une tige fonctionnelle (5) d'un piston (4) qui est logée sensiblement de manière étanche et en coulissant de manière axiale à l'intérieur dudit corps (2), l'extrémité opposée formant un bec (6) auquel est associée une canule (7) dont au moins un tronçon peut être inséré dans un conduit anatomique, en particulier dans le canal rectal d'un patient pour irriguer celui-ci avec ledit fluide (3), la canule (7) étant pourvue d'au moins une ouverture de distribution de fluide (8) qui est définie sur la surface latérale dudit tronçon, ledit instrument comprenant en outre des moyens de blocage temporaire dudit piston (4) aptes à empêcher ce dernier de coulisser de manière axiale de ladite extrémité ouverte dudit corps (2) vers ledit bec (6), **caractérisé par le fait que** lesdits moyens de blocage temporaire comprennent un élément de blocage (18) associé de manière solidaire à ladite tige (5) et qui peut venir en prise avec un élément d'arrêt (19) défini au voisinage de ladite extrémité ouverte dudit corps (2), des moyens de libération (20) étant prévus pour ledit élément de blocage (18) et ledit élément d'arrêt (19).

2. Instrument (1) selon la revendication 1, **caractérisé par le fait que** ladite canule (7) comprend un élément tubulaire (9) qui présente une extrémité ouverte et est apte à être associée audit bec (6) et dont l'extrémité opposée est fermée, ledit tronçon étant défini à proximité de ladite extrémité fermée.

3. Instrument (1) selon une ou plusieurs des revendications précédentes, **caractérisé par le fait que** ladite canule (7) comprend au moins une paire desdites ouvertures de distribution (8), définies exactement en face l'une de l'autre.

4. Instrument (1) selon la revendication 3, **caractérisé par le fait qu'**il comprend une série desdites paires (8), réparties à une certaine distance l'une de l'autre dans la direction longitudinale de ce tronçon.

5. Instrument (1) selon une ou plusieurs des revendications précédentes, **caractérisé par le fait que** ledit élément de blocage (18) comprend au moins une ailette (21) qui s'étend transversalement à partir de ladite tige (5).

6. Instrument (1) selon la revendication 5, **caractérisé par le fait que** ledit élément d'arrêt (19) comprend une dent (22) de forme annulaire définie en saillie dans la direction centripète sur la surface latérale interne dudit corps (2) et apte à venir en contact avec l'extrémité libre de ladite au moins une ailette (21).

7. Instrument (1) selon la revendication 6, **caractérisé par le fait que** lesdits moyens de libération (20) présentent l'aspect d'au moins une découpe longitudinale (23), définie dans les parois dudit corps (2) et apte à interrompre la continuité de ladite dent (22), ladite tige (5) étant capable de passer par rotation d'une configuration de blocage dudit piston (4), dans laquelle l'extrémité libre de ladite au moins une ailette (21) est en contact avec ladite dent (22), à une configuration de libération dudit piston (4), dans laquelle l'extrémité libre de ladite au moins une ailette (21) est positionnée au niveau de ladite découpe longitudinale (23).

8. Instrument (1) selon la revendication 7, **caractérisé par le fait que** lesdites ailettes (21) sont au nombre de deux et disposées complètement à l'opposé l'une de l'autre, lesdites découpes (23) étant au nombre de deux et complètement à l'opposé l'une de l'autre.

9. Instrument (1) selon une ou plusieurs des revendications 5 à 8, **caractérisé par le fait que** ladite tige (5) est fixée à une extrémité dudit piston (4) et, à l'autre extrémité, présente l'aspect d'une poignée (24) sensiblement en forme de disque, ladite au moins une ailette (21) étant située entre ladite poignée (24) et ledit piston (4).

10. Instrument (1) selon une ou plusieurs des revendications précédentes, **caractérisé par le fait qu'**il comprend un capuchon (12) susceptible d'être retiré, destiné à couvrir ladite canule (7).

11. Instrument (1) selon la revendication 10, **caractérisé par le fait que** ledit capuchon (12) est associé d'une manière amovible le long d'une ligne de moindre résistance (13) à un épaulement (14) associé étroitement audit bec (6).

12. Instrument (1) selon la revendication 10 ou 11, **caractérisé par le fait que** ledit capuchon (12) comprend au moins une paire d'anneaux d'étanchéité (17) définis sur sa surface latérale interne et aptes à constituer une limite pour ladite au moins une ouverture de distribution (8).

13. Instrument (1) selon une ou plusieurs des revendications précédentes, **caractérisé par le fait que** ledit corps (2) présente sur sa surface externe une succession de parties de préhension en saillie (26).
